# EUROPEAN PATENT APPLICATION

(11) **EP 0 900 841 A1**
(43) Date of publication of application: **10.03.1999**
(21) Application number: 97401404.5
(22) Date of filing: 18.06.1997
(51) Int. Cl.: C12N 15/12, C07K 14/725, C07K 16/28, A61K 38/17, A61K 39/395, G01N 33/53

(54) **LAG-3 splice variants**

(71) Applicant: INSTITUT NATIONAL DE LA SANTE ET DE LA RECHERCHE MEDICALE (INSERM), 75654 Paris Cédex 13 (FR); INSTITUT GUSTAVE ROUSSY, F-94805 Villejuif Cédex (FR); APPLIED RESEARCH SYSTEMS ARS HOLDING N.V., Curacao (AN)
(72) Inventor: Triebel, Frédéric, 78000 Versailles (FR); Mastrangeli, Renato, 00146 Rome (IT); Romagnani, Sergio, 50100 Firenze (IT)
(74) Representative: Hirsch, Denise

(57) **Abstract**

This invention concerns an isolated nucleotidic sequence selected from the group consisting of :
a) the nucleotidic sequences SEQ ID N° 1, SEQ ID N°3 or SEQ ID N°5 ;
b) the nucleotidic sequences which hybridize under stringent conditions to any of the sequences defined in a) and which code for a polypeptide which is a variant of the LAG-3 molecule ;
c) the nucleotidic sequences which are degenerated as results of the genetic code to the nucleotidic sequences defined in a) and b), and which code for a polypeptide which is a genetic variant of the LAG-3 molecule.

SEQUENCE LISTING

(1) GENERAL INFORMATION:

(i) APPLICANTS:
(A) NAME: Institut National de la Santé et de la
Recherche Médicale
(B) STREET: 101 rue de Tolbiac
(C) CITY: Paris
(E) COUNTRY: France
(F) POSTAL CODE (ZIP): 75013

(A) NAME: Institut Gustave Roussy
(B) STREET: 39 rue Camille Desmoulins
(C) CITY: Villejuif
(E) COUNTRY: France
(F) POSTAL CODE (ZIP): 94805
(G) TELEPHONE: 0142114211
(H) TELEFAX: 0142115300

(A) NAME : Applied Research Systems ARS Holding N.V
(B) STREET : 6 John R. Gorsiraweg PO Box 3889
(C) CITY : CURACAO
(E) COUNTRY : The Dutch West Indies

(ii) TITLE OF INVENTION: Lag-3 variants

(iii) NUMBER OF SEQUENCES: 15

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 2279 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 1:

TCAGGCTGCC TGATCTGCCC AGCTTTCCAG CTTTCCTCTG GATTCCGGCC TCTGGTCATC 60

CCTCCCCACC CTCTCTCCAA GGCCCTCTCC TGGTCTCCCT TCTTCTAGAA CCCCTTCCTC 120

CACCTCCCTC TCTGCAGAAC TTCTCCTTTA CCCCCCACCC CCCACCACTG CCCCCTTTCC 180

TTTTCTGACC TCCTTTTGGA GGGCTCAGCG CTGCCCAGAC CATAGGAGAG ATGTGGGAGG 240

CTCAGTTCCT GGGCTTGCTG TTTCTGCAGC CGCTTTGGGT GGCTCCAGTG AAGCCTCTCC 300

AGCCAGGGGC TGAGGTCCCG GTGGTGTGGG CCCAGGAGGG GGCTCCTGCC CAGCTCCCCT 360
GCAGCCCCAC AATCCCCCTC CAGGATCTCA GCCTTCTGCG AAGAGCAGGG GTCACTTGGC 420
AGCATCAGCC AGACAGTGGC CCGCCCGCTG CCGCCCCCGG CCATCCCCTG GCCCCCGGCC 480
CTCACCCGGC GGCGCCCTCC TCCTGGGGGC CCAGGCCCCG CCGCTACACG GTGCTGAGCG 540
TGGGTCCCGG AGGCCTGCGC AGCGGGAGGC TGCCCCTGCA GCCCCGCGTC CAGCTGGATG 600
AGCGCGGCCG GCAGCGCGGG GACTTCTCGC TATGGCTGCG CCCAGCCCGG CGCGCGGACG 660
CCGGCGAGTA CCGCGCCGCG GTGCACCTCA GGGACCGCGC CCTCTCCTGC CGCCTCCGTC 720
TGCGCCTGGG CCAGGCCTCG ATGACTGCCA GCCCCCCAGG ATCTCTCAGA GCCTCCGACT 780
GGGTCATTTT GAACTGCTCC TTCAGCCGCC CTGACCGCCC AGCCTCTGTG CATTGGTTCC 840
GGAACCGGGG CCAGGGCCGA GTCCCTGTCC GGGAGTCCCC CCATCACCAC TTAGCGGAAA 900
GCTTCCTCTT CCTGCCCCAA GTCAGCCCCA TGGACTCTGG GCCCTGGGGC TGCATCCTCA 960
CCTACAGAGA TGGCTTCAAC GTCTCCATCA TGTATAACCT CACTGTTCTG GGTAACTCCC 1020
CCACTCTGCT TCACATTTGA CCACAACTCC TTCCTGCCCC CCTTGTCACC TCCCCTAACT 1080
ATGGGTCCCC AAACCAGGTT CTCGGCAGCG AGTGGCCTAC GTCATTGCTG TGGGTCTCAC 1140
TGTTCGACCC CTTTATATTG CTGGCAGCCT CACAGCTGCC ATCACCCCTT CTTGCTTCTC 1200
CCGTGGCCTT CCAGCGTCAT TGCCGGCCTT CCCTCTCCTT CCGACTAAGC CCACTTGCTG 1260
GGTTTCTGAG CCTCCTCAGC TCATCACCTT ATTCTGCTCC TTAGCACTCT TATGAGCCAG 1320
ACCATCTCCT GAATTCTTCT GCCTCCCTTC CTTGCAGCCC CAGCACTCCC TCCCCACTGC 1380
AGCACCCAGC TTTAACTTTG GGTTTTCTTT TCTCTTCAGG TCTGGAGCCC CCAACTCCCT 1440
TGACAGTGTA CGCTGGAGCA GGTTCCAGGG TGGGGCTGCC CTGCCGCCTG CCTGCTGGTG 1500
TGGGGACCCG GTCTTTCCTC ACTGCCAAGT GGACTCCTCC TGGGGGAGGC CCTGACCTCC 1560
TGGTGACTGG AGACAATGGC GACTTTACCC TTCGACTAGA GGATGTGAGC CAGGCCCAGG 1620
CTGGGACCTA CACCTGCCAT ATCCATCTGC AGGAACAGCA GCTCAATGCC ACTGTCACAT 1680
TGGCAATCAT CACAGTGACT CCCAAATCCT TTGGGTCACC TGGATCCCTG GGGAAGCTGC 1740
TTTGTGAGGT GACTCCAGTA TCTGGACAAG AACGCTTTGT GTGGAGCTCT CTGGACACCC 1800
CATCCCAGAG GAGTTTCTCA GGACCTTGGC TGGAGGCACA GGAGGCCCAG CTCCTTTCCC 1860
AGCCTTGGCA ATGCCAGCTG TACCAGGGGG AGAGGCTTCT TGGAGCAGCA GTGTACTTCA 1920
CAGAGCTGTC TAGCCCAGGT GCCCAACGCT CTGGGAGAGC CCCAGGTGCC CTCCCAGCAG 1980
GCCACCTCCT GCTGTTTCTC ACCCTTGGTG TCCTTTCTCT GCTCCTTTTG GTGACTGGAG 2040
CCTTTGGCTT TCACCTTTGG AGAAGACAGT GGCGACCAAG ACGATTTTCT GCCTTAGAGC 2100
AAGGGATTCA CCCTCGCAGG CTCAGAGCAA GATAGAGGAG CTGGAGCAAG AACCGGAGCC 2160
GGAGCCGGAG CCGGAACCGG AGCCCGAGCC CGAGCCCGAG CCGGAGCAGC TCTGACCTGG 2220
AGCTGAGGCA GCCAGCAGAT CTCAGCAGCC CAGTCCAAAT AAACGTCCTG TCTAGCAGC 2279

(2) INFORMATION FOR SEQ ID NO: 2:

(i) SEQUENCE CHARACTERISTICS:
(A) LENGTH: 247 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: Homo sapiens

(xi) SEQUENCE DESCRIPTION: SEQ ID NO: 2:

Leu Gln Pro Gly Ala Glu Val Pro Val Val Trp Ala Gln Glu Gly Ala
1               5                   10                  15

Pro Ala Gln Leu Pro Cys Ser Pro Thr Ile Pro Leu Gln Asp Leu Ser
            20                  25                  30

Leu Leu Arg Arg Ala Gly Val Thr Trp Gln His Gln Pro Asp Ser Gly
        35                  40                  45

Pro Pro Ala Ala Ala Pro Gly His Pro Leu Ala Pro Gly Pro His Pro
    50                  55                  60

Ala Ala Pro Ser Ser Trp Gly Pro Arg Pro Arg Arg Tyr Thr Val Leu
65                  70                  75                  80

Ser Val Gly Pro Gly Gly Leu Arg Ser Gly Arg Leu Pro Leu Gln Pro
                85                  90                  95

Arg Val Gln Leu Asp Glu Arg Gly Arg Gln Arg Gly Asp Phe Ser Leu
            100                 105                 110

Trp Leu Arg Pro Ala Arg Arg Ala Asp Ala Gly Glu Tyr Arg Ala Ala
        115                 120                 125

Val His Leu Arg Asp Arg Ala Leu Ser Cys Arg Leu Arg Leu Arg Leu
    130                 135                 140

Gly Gln Ala Ser Met Thr Ala Ser Pro Pro Gly Ser Leu Arg Ala Ser
145                 150                 155                 160

Asp Trp Val Ile Leu Asn Cys Ser Phe Ser Arg Pro Asp Arg Pro Ala
                165                 170                 175

Ser Val His Trp Phe Arg Asn Arg Gly Gln Gly Arg Val Pro Val Arg
            180                 185                 190

Glu Ser Pro His His His Leu Ala Glu Ser Phe Leu Phe Leu Pro Gln
        195                 200                 205

Val Ser Pro Met Asp Ser Gly Pro Trp Gly Cys Ile Leu Thr Tyr Arg
    210                 215                 220

Asp Gly Phe Asn Val Ser Ile Met Tyr Asn Leu Thr Val Leu Gly Asn
225                 230                 235                 240

Ser Pro Thr Leu Leu His Ile

                245

(2) INFORMATION FOR SEQ ID NO: 3:

(i) SEQUENCE CHARACTERISTICS:
(A) LENGTH: 1629 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 3:

TCAGGCTGCC TGATCTGCCC AGCTTTCCAG CTTTCCTCTG GATTCCGGCC TCTGGTCATC 60

CCTCCCCACC CTCTCTCCAA GGCCCTCTCC TGGTCTCCCT TCTTCTAGAA CCCCTTCCTC 120

CACCTCCCTC TCTGCAGAAC TTCTCCTTTA CCCCCCACCC CCCACCACTG CCCCCTTTCC 180

TTTTCTGACC TCCTTTTGGA GGGCTCAGCG CTGCCCAGAC CATAGGAGAG ATGTGGGAGG 240

CTCAGTTCCT GGGCTTGCTG TTTCTGCAGC CGCTTTGGGT GGCTCCAGTG AAGCCTCTCC 300

AGCCAGGGGC TGAGGTCCCG GTGGTGTGGG CCCAGGAGGG GGCTCCTGCC CAGCTCCCCT 360

GCAGCCCCAC AATCCCCCTC CAGGATCTCA GCCTTCTGCG AAGAGCAGGG GTCACTTGGC 420

AGCATCAGCC AGACAGTGGC CCGCCCGCTG CCGCCCCCGG CCATCCCCTG GCCCCCGGCC 480

CTCACCCGGC GGCGCCCTCC TCCTGGGGGC CCAGGCCCCG CCGCTACACG GTGCTGAGCG 540

TGGGTCCCGG AGGCCTGCGC AGCGGGAGGC TGCCCCTGCA GCCCCGCGTC CAGCTGGATG 600

AGCGCGGCCG GCAGCGCGGG GACTTCTCGC TATGGCTGCG CCCAGCCCGG CGCGCGGACG 660

CCGGCGAGTA CCGCGCCGCG GTGCACCTCA GGGACCGCGC CCTCTCCTGC CGCCTCCGTC 720

TGCGCCTGGG CCAGGCCTCG ATGACTGCCA GCCCCCCAGG ATCTCTCAGA GCCTCCGACT 780

GGGTCATTTT GAACTGCTCC TTCAGCCGCC CTGACCGCCC AGCCTCTGTG CATTGGTTCC 840

GGAACCGGGG CCAGGGCCGA GTCCCTGTCC GGGAGTCCCC CCATCACCAC TTAGCGGAAA 900

GCTTCCTCTT CCTGCCCCAA GTCAGCCCCA TGGACTCTGG GCCCTGGGGC TGCATCCTCA 960

CCTACAGAGA TGGCTTCAAC GTCTCCATCA TGTATAACCT CACTGTTCTG GGTCTGGAGC 1020

CCCCAACTCC CTTGACAGTG TACGCTGGAG CAGGTTCCAG GGTGGGGCTG CCCTGCCGCC 1080

TGCCTGCTGG TGTGGGGACC CGGTCTTTCC TCACTGCCAA GTGGACTCCT CCTGGGGGAG 1140

GCCCTGACCT CCTGGTGACT GGAGACAATG GCGACTTTAC CCTTCGACTA GAGGATGTGA 1200

GCCAGGCCCA GGCTGGGACC TACACCTGCC ATATCCATCT GCAGGAACAG CAGCTCAATG 1260

CCACTGTCAC ATTGGCAATC ATCACAGGTG CCCAACGCTC TGGGAGAGCC CCAGGTGCCC 1320

TCCCAGCAGG CCACCTCCTG CTGTTTCTCA CCCTTGGTGT CCTTTCTCTG CTCCTTTTGG 1380

TGACTGGAGC CTTTGGCTTT CACCTTTGGA GAAGACAGTG GCGACCAAGA CGATTTTCTG 1440

CCTTAGAGCA AGGGATTCAC CCTCCGCAGG CTCAGAGCAA GATAGAGGAG CTGGAGCAAG 1500

AACCGGAGCC GGAGCCGGAG CCGGAACCGG AGCCCGAGCC CGAGCCCGAG CCGGAGCAGC 1560

TCTGACCTGG AGCTGAGGCA GCCAGCAGAT CTCAGCAGCC CAGTCCAAAT AAACGTCCTG 1620

TCTAGCAGC 1629

(2) INFORMATION FOR SEQ ID NO: 4:

(i) SEQUENCE CHARACTERISTICS:
(A) LENGTH: 422 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: Homo sapiens

(xi) SEQUENCE DESCRIPTION: SEQ ID NO: 4:

Leu Gln Pro Gly Ala Glu Val Pro Val Val Trp Ala Gln Glu Gly Ala
1               5                   10                  15

Pro Ala Gln Leu Pro Cys Ser Pro Thr Ile Pro Leu Gln Asp Leu Ser
            20                  25                  30

Leu Leu Arg Arg Ala Gly Val Thr Trp Gln His Gln Pro Asp Ser Gly
        35                  40                  45

Pro Pro Ala Ala Ala Pro Gly His Pro Leu Ala Pro Gly Pro His Pro
    50                  55                  60

Ala Ala Pro Ser Ser Trp Gly Pro Arg Pro Arg Arg Tyr Thr Val Leu
65                  70                  75                  80

Ser Val Gly Pro Gly Gly Leu Arg Ser Gly Arg Leu Pro Leu Gln Pro
                85                  90                  95

Arg Val Gln Leu Asp Glu Arg Gly Arg Gln Arg Gly Asp Phe Ser Leu
            100                 105                 110

Trp Leu Arg Pro Ala Arg Arg Ala Asp Ala Gly Glu Tyr Arg Ala Ala
        115                 120                 125

Val His Leu Arg Asp Arg Ala Leu Ser Cys Arg Leu Arg Leu Arg Leu
    130                 135                 140

Gly Gln Ala Ser Met Thr Ala Ser Pro Pro Gly Ser Leu Arg Ala Ser
145                 150                 155                 160

Asp Trp Val Ile Leu Asn Cys Ser Phe Ser Arg Pro Asp Arg Pro Ala
                165                 170                 175

Ser Val His Trp Phe Arg Asn Arg Gly Gln Gly Arg Val Pro Val Arg
            180                 185                 190

Glu Ser Pro His His His Leu Ala Glu Ser Phe Leu Phe Leu Pro Gln
        195                 200                 205

Val Ser Pro Met Asp Ser Gly Pro Trp Gly Cys Ile Leu Thr Tyr Arg
    210                 215                 220

Asp Gly Phe Asn Val Ser Ile Met Tyr Asn Leu Thr Val Leu Gly Leu
225                 230                 235                 240

Glu Pro Pro Thr Pro Leu Thr Val Tyr Ala Gly Ala Gly Ser Arg Val
                245                 250                 255

Gly Leu Pro Cys Arg Leu Pro Ala Gly Val Gly Thr Arg Ser Phe Leu
            260                 265                 270

Thr Ala Lys Trp Thr Pro Pro Gly Gly Gly Pro Asp Leu Leu Val Thr
        275                 280                 285

Gly Asp Asn Gly Asp Phe Thr Leu Arg Leu Glu Asp Val Ser Gln Ala
    290                 295                 300

Gln Ala Gly Thr Tyr Thr Cys His Ile His Leu Gln Glu Gln Gln Leu
305                 310                 315                 320

Asn Ala Thr Val Thr Leu Ala Ile Ile Thr Gly Ala Gln Arg Ser Gly
                325                 330                 335

Arg Ala Pro Gly Ala Leu Pro Ala Gly His Leu Leu Leu Phe Leu Thr
            340                 345                 350

Leu Gly Val Leu Ser Leu Leu Leu Leu Val Thr Gly Ala Phe Gly Phe
        355                 360                 365

His Leu Trp Arg Arg Gln Trp Arg Pro Arg Arg Phe Ser Ala Leu Glu
    370                 375                 380

Gln Gly Ile His Pro Pro Gln Ala Gln Ser Lys Ile Glu Glu Leu Glu
385                 390                 395                 400

Gln Glu Pro Glu Pro Glu Pro Glu Pro Glu Pro Glu Pro Glu Pro Glu
                405                 410                 415

Pro Glu Pro Glu Gln Leu
            420

(2) INFORMATION FOR SEQ ID NO: 5:

(i) SEQUENCE CHARACTERISTICS:
(A) LENGTH: 1468 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 5:

TCAGGCTGCC TGATCTGCCC AGCTTTCCAG CTTTCCTCTG GATTCCGGCC TCTGGTCATC      60

CCTCCCCACC CTCTCTCCAA GGCCCTCTCC TGGTCTCCCT TCTTCTAGAA CCCCTTCCTC     120

CACCTCCCTC TCTGCAGAAC TTCTCCTTTA CCCCCCACCC CCCACCACTG CCCCCTTTCC     180

TTTTCTGACC TCCTTTTGGA GGGCTCAGCG CTGCCCAGAC CATAGGAGAG ATGTGGGAGG     240

CTCAGTTCCT GGGCTTGCTG TTTCTGCAGC CGCTTTGGGT GGCTCCAGTG AAGCCTCTCC     300

AGCCAGGGGC TGAGGTCCCG GTGGTGTGGG CCCAGGAGGG GGCTCCTGCC CAGCTCCCCT     360

GCAGCCCCAC AATCCCCCTC CAGGATCTCA GCCTTCTGCG AAGAGCAGGG GTCACTTGGC     420

AGCATCAGCC AGACAGTGGC CCGCCCGCTG CCGCCCCCGG CCATCCCCTG GCCCCCGGCC 480

CTCACCCGGC GGCGCCCTCC TCCTGGGGGC CCAGGCCCCG CCGCTACACG GTGCTGAGCG 540

TGGGTCCCGG AGGCCTGCGC AGCGGGAGGC TGCCCCTGCA GCCCCGCGTC CAGCTGGATG 600

AGCGCGGCCG GCAGCGCGGG GACTTCTCGC TATGGCTGCG CCCAGCCCGG CGCGCGGACG 660

CCGGCGAGTA CCGCGCCGCG GTGCACCTCA GGGACCGCGC CCTCTCCTGC CGCCTCCGTC 720

TGCGCCTGGG CCAGGCCTCG ATGACTGCCA GCCCCCCAGG ATCTCTCAGA GCCTCCGACT 780

GGGTCATTTT GAACTGCTCC TTCAGCCGCC CTGACCGCCC AGCCTCTGTG CATTGGTTCC 840

GGAACCGGGG CCAGGGCCGA GTCCCTGTCC GGGAGTCCCC CCATCACCAC TTAGCGGAAA 900

GCTTCCTCTT CCTGCCCCAA GTCAGCCCCA TGGACTCTGG GCCCTGGGGC TGCATCCTCA 960

CCTACAGAGA TGGCTTCAAC GTCTCCATCA TGTATAACCT CACTGTTCTG GGTCTGGAGC 1020

CCCCAACTCC CTTGACAGTG TACGCTGGAG CAGGTTCCAG GGTGGGGCTG CCCTGCCGCC 1080

TGCCTGCTGG TGTGGGGACC CGGTCTTTCC TCACTGCCAA GTGGACTCCT CCTGGGGGAG 1140

GCCCTGACCT CCTGGTGACT GGAGACAATG GCGACTTTAC CCTTCGACTA GAGGATGTGA 1200

GCCAGGCCCA GGCTGGGACC TACACCTGCC ATATCCATCT GCAGGAACAG CAGCTCAATG 1260

CCACTGTCAC ATTGGCAATC ATCACAGGTC AGCCTCAGGT GGGAAAGGAG TAGCTGCCCT 1320

CCCAGGGTAG AAAGGACAGG GAGGAAGGGC TGGCAGGGCA AAGACTAGGC AAACCCACCC 1380

TGTGATGCCA GGCCACTGGG CACAAGTTCC AGAGCCTGCC CATCTCGGCC CCCACTTTTC 1440

TCACCCCCAT AATAAAGAAA CGAAACTG 1468

(2) INFORMATION FOR SEQ ID NO: 6:

(i) SEQUENCE CHARACTERISTICS:
(A) LENGTH: 338 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: Homo sapiens

(xi) SEQUENCE DESCRIPTION: SEQ ID NO: 6:

Leu Gln Pro Gly Ala Glu Val Pro Val Val Trp Ala Gln Glu Gly Ala
1               5                   10                  15

Pro Ala Gln Leu Pro Cys Ser Pro Thr Ile Pro Leu Gln Asp Leu Ser
            20                  25                  30

Leu Leu Arg Arg Ala Gly Val Thr Trp Gln His Gln Pro Asp Ser Gly
        35                  40                  45

Pro Pro Ala Ala Ala Pro Gly His Pro Leu Ala Pro Gly Pro His Pro
    50                  55                  60

Ala Ala Pro Ser Ser Trp Gly Pro Arg Pro Arg Arg Tyr Thr Val Leu
65                  70                  75                  80

Ser Val Gly Pro Gly Gly Leu Arg Ser Gly Arg Leu Pro Leu Gln Pro
                85                  90                  95

Arg Val Gln Leu Asp Glu Arg Gly Arg Gln Arg Gly Asp Phe Ser Leu
            100                 105                 110

Trp Leu Arg Pro Ala Arg Arg Ala Asp Ala Gly Glu Tyr Arg Ala Ala
        115                 120                 125

Val His Leu Arg Asp Arg Ala Leu Ser Cys Arg Leu Arg Leu Arg Leu
    130                 135                 140

Gly Gln Ala Ser Met Thr Ala Ser Pro Pro Gly Ser Leu Arg Ala Ser
145                 150                 155                 160

Asp Trp Val Ile Leu Asn Cys Ser Phe Ser Arg Pro Asp Arg Pro Ala
                165                 170                 175

Ser Val His Trp Phe Arg Asn Arg Gly Gln Gly Arg Val Pro Val Arg
            180                 185                 190

Glu Ser Pro His His His Leu Ala Glu Ser Phe Leu Phe Leu Pro Gln
        195                 200                 205

Val Ser Pro Met Asp Ser Gly Pro Trp Gly Cys Ile Leu Thr Tyr Arg
    210                 215                 220

Asp Gly Phe Asn Val Ser Ile Met Tyr Asn Leu Thr Val Leu Gly Leu
225                 230                 235                 240

Glu Pro Pro Thr Pro Leu Thr Val Tyr Ala Gly Ala Gly Ser Arg Val
                245                 250                 255

Gly Leu Pro Cys Arg Leu Pro Ala Gly Val Gly Thr Arg Ser Phe Leu
            260                 265                 270

Thr Ala Lys Trp Thr Pro Pro Gly Gly Gly Pro Asp Leu Leu Val Thr
        275                 280                 285

Gly Asp Asn Gly Asp Phe Thr Leu Arg Leu Glu Asp Val Ser Gln Ala
    290                 295                 300

Gln Ala Gly Thr Tyr Thr Cys His Ile His Leu Gln Glu Gln Gln Leu
305                 310                 315                 320

Asn Ala Thr Val Thr Leu Ala Ile Ile Thr Gly Gln Pro Gln Val Gly
                325                 330                 335

Lys Glu

(2) INFORMATION FOR SEQ ID NO: 7:

    (i) SEQUENCE CHARACTERISTICS:
        (A) LENGTH: 29 base pairs
        (B) TYPE: nucleic acid
        (C) STRANDEDNESS: single
        (D) TOPOLOGY: linear

    (ii) MOLECULE TYPE: cDNA

(xi) SEQUENCE DESCRIPTION: SEQ ID NO: 7:

TCTCTCAGAG CCTCCGATGG GTCATTTTG 29

(2) INFORMATION FOR SEQ ID NO: 8:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 8:

TCCTGCAGAT GGATATGGCA GGTGTAGGTC 30

(2) INFORMATION FOR SEQ ID NO: 9:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 9:

CCTGGGCCAG GCCTCGATGA C 21

(2) INFORMATION FOR SEQ ID NO: 10:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 10:

CCCCACTCTG CTTCACATTT 20

(2) INFORMATION FOR SEQ ID NO: 11:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 11:

GGCCCTGGAT CCGGACCTAA TTTTTTTTTT TTTTTTT 37

(2) INFORMATION FOR SEQ ID NO: 12:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 12:

GGCCCTGGAT CCGGACCTAA 20

(2) INFORMATION FOR SEQ ID NO: 13:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 13:

TATAGGATCC GGTGCCCAGA CCATAGGAGA GATG 34

(2) INFORMATION FOR SEQ ID NO: 14:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 14:

GGCGTTCACG TGGTTGGGCA CCTGTGATGA TT 32

(2) INFORMATION FOR SEQ ID NO: 15:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 15:

TCACCTACTC GAGAAAAGTG GGGGCCGAGA T 31

## Description

The present invention relates to the identification of differentially spliced variants of the LAG-3 molecule and their use as immunomodulators.

It is now recognized that the proteins encoded by MHC Class II region are involved in many aspects of immune recognition, including the interaction between different lymphoid cells such as lymphocytes and antigen presenting cells. Different observations have also shown that other mechanisms which do not take place via CD4 participate in the effector function of T helper lymphocytes.

The lymphocyte activation gene 3 (LAG-3) expressed in human activated T and NK cells encodes a 498 amino-acids (aa) type I membrane protein with four extracellular immunoglobulin superfamily (IgSF) domains (1). Analysis of this sequence revealed notable patches of identity with stretches of aa sequences found at the corresponding positions in CD4, although the overall aa sequence homology with human CD4 is barely above background level (approximately 20 % sequence identity). There are also some internal sequence homologies in the LAG-3 molecule between domains 1 (D1) and 3 (D3) as well as between domains 2 (D2) and 4 (D4) suggesting that LAG-3 has evolved like CD4 by gene duplication from a preexisting 2 IgSF structure (1). In addition, LAG-3 and CD4 genes are located in a very close proximity on the distal part of the short arm of chromosome 12 (2, B. Huard, P. Gaulard, F. Faure, T. Hercend, F. Triebel, Immunogenetics 39, 213, 1994). LAG-3 and CD4 can therefore be regarded as evolutionary "first cousins" within the IgSF (2).

Using a quantitative cellular adhesion assay, the authors of the present invention previously showed that rosette formation between LAG-3 transfected Cos-7 cells and MHC class II⁺ B lymphocytes was specifically dependent on LAG-3/MHC class II interaction (3). A direct an specific binding of LAG-3 to various human class II molecules (including different alleles and isotypes), as well as to murine and monkey class II molecules has also been observed with a LAG-3Ig fusion protein (4). This dimeric LAG-3Ig recombinant globulin binds MHC class II monomorphic residues with a much higher avidity (Kd = 60 nM at 37° C) than CD4Ig (5); LAG-3Ig is indeed able to block CD4/MHC class II interaction in an intercellular adhesion assay (5).

The role of LAG-3/class II molecule interaction has been investigated using LAG-3 specific monoclonal antibodies (mAb) (6) and LAG-3Ig molecules (7). This interaction leads to downregulation of T cell clone activation. Productive LAG-3/MHC class II interaction is mediated through T-T cell contacts, presumably via negative MHC class II signaling into T cells. Overall, LAG-3 is expressed only affer lymphocyte activation *in vitro* as well as *in vivo* (6) and hence does not play a role in the induction phase of the response, in contrast to CD4. In addition, mAb blocking experiments have shown that LAG-3 does not participate in the recognition phase of MHC class II-restricted CD4⁺ T cell clones. The functional role of LAG-3 is therefore strikingly different from that of the other MHC ligands, CD4 and CD8. It is currently thought that T cell MHC class II molecules have a role similar to CTLA-4 following LAG-3 binding (4), i.e. induction of clonal deletion of previously activated T cells (8).

More recently, LAG-3 has been found to be preferentially expressed and released by activated Th1 cells, i.e. T helper cells producing IFN-γ and TNF, and to be upregulated by IL-12, a powerful Th1-inducing cytokine (F. Annunziato, R. Manetti, L. Tomasévic, MG. Giudizi, R. Biagiotti, V. Giannò, P. Germano, C. Mavilia, E. Maggi and S. Romagnani, FASEB J. 10, 769-775, 1996). High levels of soluble LAG-3 (sLAG-3) were also found in the serum of patients with relapsing multiple sclerosis (MS) (ibidem) and of a few patients with systemic lupus erythematosus (S. Romagnani, Clin. Immunol. Immunopath. 80, 225-235, 1996). These findings suggest that LAG-3 could represent an useful diagnostic marker for Th1-mediated immune diseases, such as Hashimoto's thyroiditis, type I diabetes mellitus, multiple sclerosis, Chron's disease, rheumatoid arthritis, acute allograft rejection and acute graft-versus-host disease (GVHD). On the other hand, the presence of high levels of sLAG-3 in the serum of MS patients and possibly of other patients suffering of the above diseases, supports a naturally occurring protective role for sLAG-3, as it could compete with the membrane-bound form and thus block the LAG-3-mediated immune responses.

The authors of the present invention have investigated whether other forms of LAG-3 are expressed, due to alternative splicing of the nuclear transcript.

Most eukaryotic protein encoding genes contain the sequences present in the corresponding mature mRNA in discontinuous DNA fragments (exons) interspersed among sequences (introns) that do not form a part of the mature mRNA. The primary transcripts of these genes thus contain both sequences corresponding to exons and sequences corresponding to introns. The intron sequences are subsequently excised by a nuclear multistep process, known as pre-mRNA splicing. Namely, introns are demarcated by consensus sequences at their 5'(donor) and 3'(acceptor) boundaries. The splicing process comprises cleavage at the acceptor site with concomitant ligation of the 5' and 3' exons.

In most cases, the exons present in a gene are incorporated into one mature mRNA through an invariant ligation of consecutive pairs of donor ans acceptor splice sites, providing a single gene product.

In some cases however, the same gene contain alternate splice sites which may lead to different transcripts. The mechanism regulating this alternative splicing are poorly understood, but at least for some genes, it is known to be cell and developmental specific.

The alternative splicing leads to the production of multiple protein isoforms from a single gene. It is one of the molecular process responsible for generating the protein diversity.

More than 50 genes are currently known to generate protein diversity through the use of alternative splicing. This mechanism is particularly common and elaborated among the contractile protein genes.

The authors of the present invention have discovered three new differentially spliced LAG-3 variants, respectively referred as LAG-3V1, LAG-3V2, and LAG-3V3.

LAG-3VI encodes a soluble 36 kD protein containing 8 new aminoacid residues after the D2 domain, LAG-3V2 encodes a transmembrane protein of 61 kD which does not contain domain D4, and LAG-3V3 encodes a soluble 52 kD protein containing 8 new aminoacid residues after domain D3.

These variants provide new approaches for studying both the regulation of the LAG-3 gene and the biological function of the protein, and are useful for the manufacture of new immunomodulator compounds, especially compounds which mimic the biological function of LAG-3 or which can act as agonists or antagonists of the interaction between LAG-3 and the MHC class II molecules.

The subject of the present invention is thus an isolated nucleotidic sequence selected from the group consisting of:
a) the nucleotidic sequences SEQ ID N° 1, SEQ ID N° 3 or SEQ ID N°5;
b) the nucleotidic sequences which hybridize under stringent conditions to any of the sequences defined in a) and which code for a polypeptide which is a variant of the LAG-3 molecule;
c) the nucleotidic sequences which are degenerated as results of the genetic code to the nucleotidic sequences defined in a) and b), and which code for a polypeptide which is a genetic variant of the LAG-3 molecule.

The present invention is also directed to the purified polypeptides encoded by the nucleotidic sequences defined above. Said polypeptides are designated hereinafter as "LAG-3 variants". The present invention is also relating to pharmaceutical compositions comprising a LAG-3 variant polypeptide. These compositions are useful for treating immune-related pathologies, in particular Th1-dependent diseases such as Hashimoto's thyroiditis, type I diabetes mellitus, multiple sclerosis, Chron's disease, rheumatoid arthritis, acute allograft rejection, acute GVHD, Grave's ophtalmopathy, cerebral malaria, Lyme arthritis, reactive arthritis (Yersinia-induced), HCV-induced chronic hepatitis, primary sclerosing colangitis, contact dermatitis, unexplained recurrent abortion, aplastic anaemia, and Helicobacter pilori-induced gastric antritis.

The present invention also relates to the use of the LAG-3 variants for the manufacture of immunomodulator compounds. Such compounds can mimic or alter the biological function of LAG-3 and/or LAG-3 variants, inducing therefore some modifications of the cellular interactions involving the participation of LAG-3 or its variants.

The present invention is also directed to poly- or monoclonal antibodies, Fab, Fab' and F(ab')₂ or Fv fragments thereof, directed to a specific epitope of one of the above-defined LAG-3 variants. The use of these specific antibodies for purifying said variants or for preparing therapeutic or diagnostic compositions, as well as the resulting compositions, are also comprised within the scope of the present invention.

The present invention further provides a therapeutic method for treating immune-related pathologies comprising the administration to a patient of a LAG-3 variant in an efficient quantity or a composition comprising said variant as an active ingredient.

The invention is also relating to a therapeutic method for treating immune-related pathologies comprising the administration to a patient of an anti-LAG-3 variant- antibody as defined here-above, or a therapeutic composition containing said antibody as active ingredient.

In a preferred embodiment, the invention is directed to an isolated nucleotide sequence selected from the group consisting of SEQ ID N° 1, SEQ ID N° 3, SEQ ID N° 5 or the fully complementary sequences thereof.

The invention also relates to a purified polypeptide which is resulting from the expression of one of the nucleotidic sequences of the invention. Preferably, the polypeptide is selected from the group consisting of LAG-3V1, LAG-3V2 and LAG-3V3, respectively encoded by the sequences SEQ ID N° 1, SEQ ID N° 3 and SEQ ID N° 5 and respectively corresponding to the sequences SEQ ID N° 2, SEQ ID N° 4 and SEQ ID N° 6.

The polypeptides according to the invention can be obtained by any of the standard methods of purification of membrane or soluble proteins, by peptide synthesis or by application of genetic engineering techniques. Said techniques comprise the insertion of a nucleotidic sequence coding for one of the peptide of the invention into an expression vector, such as a plasmid, and the transformation of host cells with this expression vector, by any of the methods available to the skilled person, like for instance electroporation.

Advantageously, the nucleotidic sequences of the invention are obtained by RT-PCR (Reverse Transcriptase Polymerase Chain Reaction) of the corresponding LAG-3 variant cDNA. Briefly, total RNA is extracted from activated human peripheral blood lymphocytes (PBL), and is reverse-transcribed and amplified using a specific pair of oligonucleotides primers. In the case of the present invention, the primers are consisting of a sense primer and an antisense primer which are located at two specific positions on the LAG-3 sequence which will be described in more details in the following examples. The resulting cDNA is then amplified by enzymatic amplification and can be subcloned in an appropriate host cell.

The present invention also relates to expression vectors comprising a nucleotidic sequence coding for a polypeptide according to the invention and host cells transformed with these vectors.

An "expression vector" refers to a replicable DNA construct used either to amplify or to express DNA which encodes one of the polypeptides of the invention.

Host cells may be prokaryotic or eukaryotic, including but not limited to bacteria, yeasts, insect cells, mammalian cells, including cell lines which are commercially available.

The present invention is also directed to anti-LAG-3 variants antibodies or fragments thereof, as defined above. Optionally, these antibodies or their fragments can be linked to a marker (radioisotope, fluorescent dye, enzymatic marker...) or to a therapeutically active molecule, which is for example a cytotoxic compound.

The polyclonal antibodies may be prepared according to well-known methods, such as that described by BENEDICT A.A. et al. ( ). Methods of production of monoclonal antibodies are well known from the prior art, especially the one described by KOHLER and MILSTEIN. This method, together with variants thereof, are described by YELTON et al ( ).

These antibodies can be used in a method for purifying a polypeptide according to the invention, or for a dosage or identification method. Said method is for example, but not limited to, a radio-immunological method of the RIA or IRMA type, an immuno-enzymatic method, like the ELISA method, or a direct or indirect immunofluorescence method.

The invention also includes the use of said antibodies for the manufacture of therapeutic compositions able to block the activity of LAG-3 variants, i.e. having immunomodulatory activities, such as induction of the maturation, differentiation, proliferation and/or function of cells expressing a LAG-3 variant, e.g. activated T and NK cells.

The antibodies to LAG-3 variants may be used as potentiators of vaccines or immunostimulants in immunosuppressed patients, such as patients infected with HIV or treated with immunosuppressant substances.

The therapeutic compositions according to the present invention comprise soluble LAG-3 variant proteins or antibodies as defined above, as well as a pharmaceutically acceptable vehicle. These compositions may be formulated according to the usual techniques. The vehicle can vary in form in accordance with the chosen administration route: oral, parenteral, sublingal, rectal or nasal.

For the compositions for parenteral administration, the vehicle will generally comprise sterile water as well as other possible ingredients promoting the solubility of the composition or its ability to be stored. The parenteral administration route can consist of intravenous, intramuscular or subcutaneous injections.

The therapeutic composition can be of the sustained-release type, in particular for long-term treatments, for example in autoimmune diseases. The dose to be administered depends on the subject to be treated, in particular on the capacity of his/her immune system to achieve the desired degree of protection. The precise amounts of active ingredient to be administered may be readily determined by the practitioner who will initiate the treatment.

The therapeutic compositions according to the invention can comprise, in addition to soluble LAG-3 variants or the antibodies according to the invention, another active ingredient, where appropriate, bound via a chemical bond to LAG-3 variant or to an antibody according to the invention. As an example, there may be mentioned soluble LAG-3 variant proteins according to the invention fused to a toxin, for example ricin or diphteria anatoxin, capable of binding to MHC Class II molecules and of killing the target cells, for example Ieukaemic or melanoma cells, or fused to a radioisotope.

The examples which follow, together with the attached reference figures, will illustrate the invention in greater detail.

### LEGENDS TO FIGURES

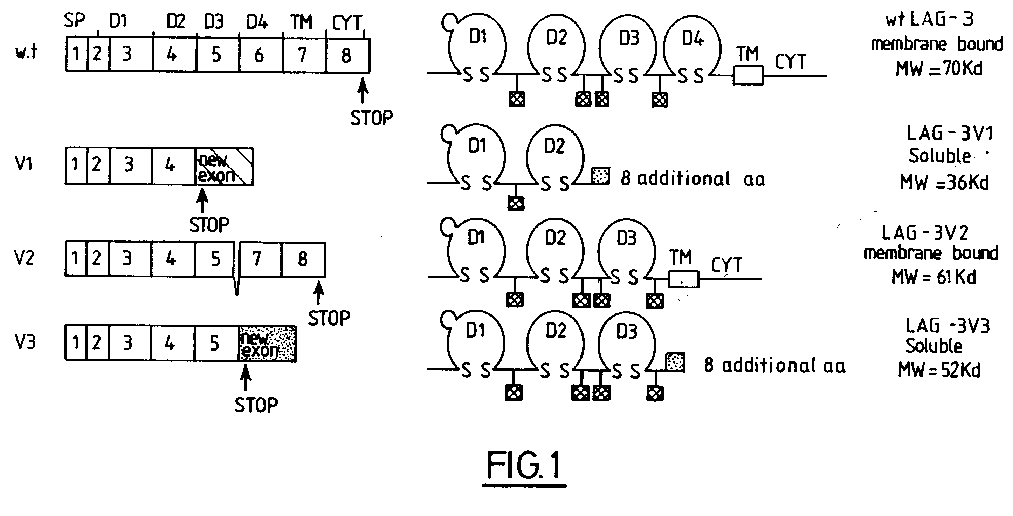

- Fig. 1 represents the schematic structure of wild type (wt) LAG-3 and its variants V1, V2 and V3. LAG-3V1 derives from the retention of intron 4, i.e. cleavage at the donor and acceptor sites flanking intron 4 does not occur. An in-frame stop codon located after 8 codons in the retained intron 4 leads to a truncated soluble LAG-3V1 protein, containing D1, D2 and 8 new amino acid residues.
   LAG-3V2 lacks exon 6, due to the ligation of the donor site on intron 5 to the acceptor site on intron 6. As no shift of the reading frame occurs, the resulting LAG-3V2 protein is a transmembrane protein which does not contain the D4 domain.
   LAG-3V3 derives from cleavage of the nuclear transcript at a different polyadenylation site located ∼170 bp downstream to the 5' end of intron 5. The retained intron 5 sequence contains an in-frame stop codon. The resulting mRNA encodes a truncated soluble protein which contains D1, D2, D3 and 8 new amino acid residues.
- Fig. 2 is a schematic representation of the intron/exon organization of the LAG-3 gene. Splicing events which result in the generation of RNA transcripts encoding either wtLAG-3, LAG-3V1, LAG-3V2 or LAG-3V3 are indicated by dotted lines.
   SP: signal peptide; D1-D4: IgSF domains 1-4; TM: transmembrane sequence; CYT: cytoplasmic domain; I1-I9: intron 1-9.
   New exons in LAG-3V1 and LAG-3V3 are shown as shaded boxes.
- Fig. 3 is a schematic representation of wtLAG-3 and LAG-3V1. The location of the primers and probes used for the isolation of LAG-3V1 is indicated by arrows. DNA fragments amplified using the specific primers are shown by single lines. The size of the fragments obtained by RT-PCR is indicated.
- Fig. 4 shows a Southern blot analysis of wtLAG-3 and LAG-3V1 cDNA fragments amplified by RT-PCR with primers F459 and R460.
   a) ethidium bromide stained gel. b) blot hybridized with a LAG-3 specific cDNA probe. The lower band derives from wtLAG-3 while the upper one, evident only after hybridization, derives from LAG-3V1.
- Fig. 5 shows a Southern blot analysis of LAG-3V1 cDNA fragments amplified by RT-PCR with primers F176 and R460. a) ethidium bromide stained gel. The major band derives from wtLAG-3. b) blot hybridized with the LAG-3 intron 4 specific oligoprobe I4. The upper band derives from LAG-3V1 while the lower one is a wtLAG-3/LAG-3V1 heteroduplex.
- Fig. 6 is a schematic representation of wtLAG-3, LAG-3V2 and LAG-3V3. The location of the primers and probes used for the isolation of LAG-3V2 and LAG-3V3 is indicated by arrows. DNA fragments amplified using the specific primers are shown by single lines. The size of the fragments obtained by RT-PCR is indicated.
- Fig. 7 shows a Southern blot analysis of wtLAG-3, LAG-3V2 and LAG-3V3 cDNA fragments amplified by RT-PCR with primers F176 and R401.
   a) ethidium bromide stained gel. b) blot hybridized with the LAG-3D2 specific oligoprobe F459. The upper band derives from wtLAG-3 while the 780 and 940 bp bands derive from LAG-3V2 and LAG-3V3, respectively.
- Fig. 8 shows the agarose gel electrophoresis pattern of RT-PCR products obtained with LAG-3V2 and LAG-3V3 specific primers, i.e. 173/V2R and 173/V3R, respectively.
- Fig. 9 shows the Western blotting patterns of PHA-blasts with anti LAG-3 in mAbs.

### EXAMPLES

### Example I:

### Cloning of human LAG-3 variant 1 (LAG-3V1) by RT-PCR on human PBMC (Peripheral Blood Mononuclear Cells)

### - RNA extraction and RT-PCR:

PBMC were isolated by Ficoll-Hypaque density gradient centrifugation and activated with 1 µg/ml PHA and 100 U/ml IL-2 for 48 hr at 37°C, 5% CO₂. mRNA was extracted by the Oligotex Direct mRNA kit (Ouiagen Inc., Chatsworth, CA, USA.) and reverse-transcribed with an oligo(dT) primer, using a first strand synthesis kit (RT-PCR kit, Stratagen, La Jolla, CA, USA).

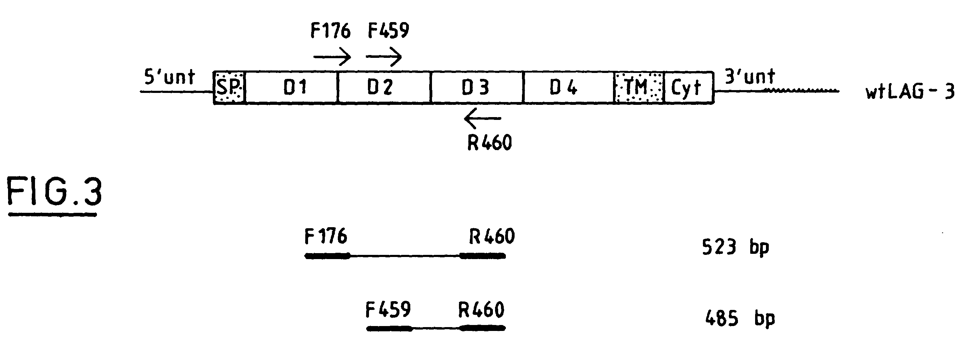

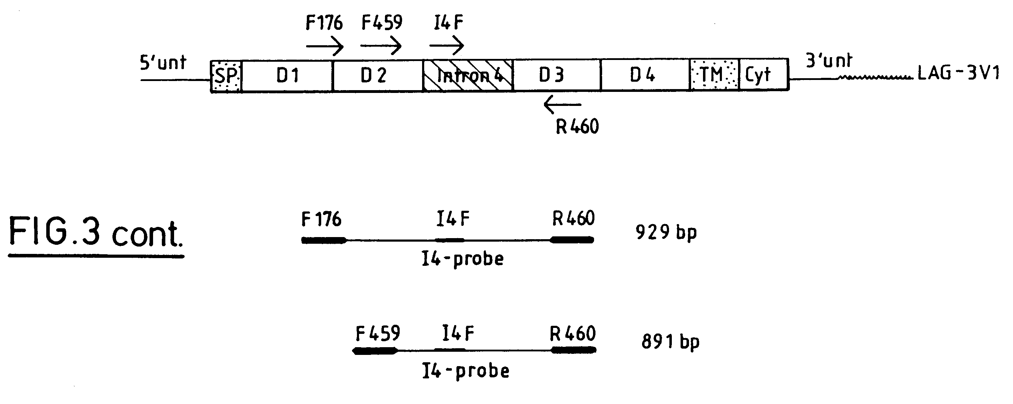

Amplification of cDNA was performed with 2.5 U Taq polymerase and 100 ng each of forward primer F459 (5' TCTCTCAGAGCCTCCGATGGGTCATTTTG 3') (SEQ ID N° 7) and reverse primer R460 (5' TCCTGCAGATGGATATGGCAGGTGTAGGTC 3') (SEQ ID N° 8) which anneal to nucleotides 762-791 and 1217-1246 of LAG-3 sequence, respectively (Fig. 3). Thirty PCR cycles were performed, each cycle including denaturation at 94°C for 1 min, annealing at 66°C for 1 min, and extension at 72°C for 2 min.

### - Analysis of amplified DNA by Southern blotting :

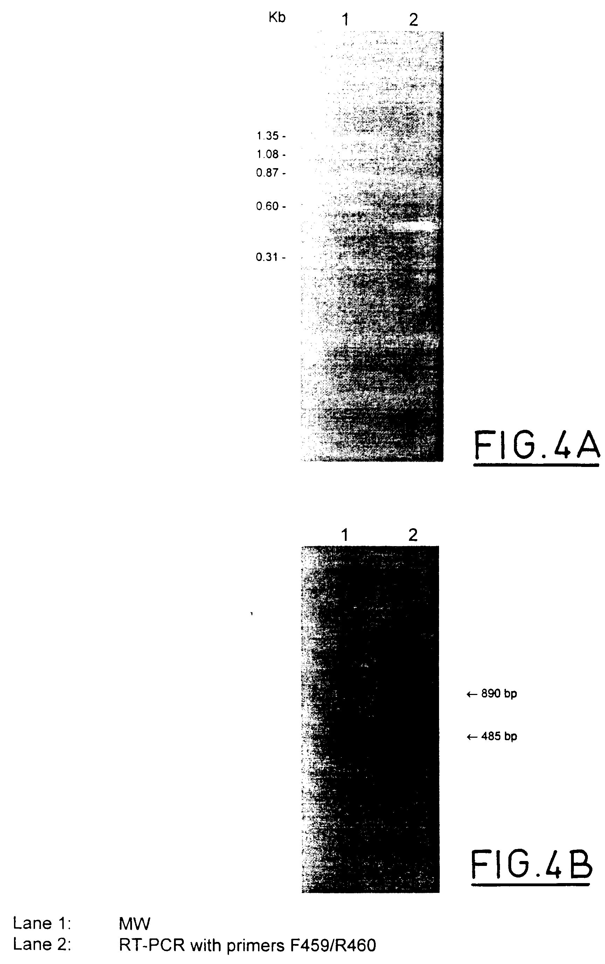

A 10 µl aliquot of the amplified DNA was fractionated on a 2 % agarose gel. The expected 485 bp LAG-3 fragment was observed (Fig. 4a). After blotting onto nitrocellulose membrane filter and hybridization with a ³²P-labelled LAG-3D1D2 specific cDNA probe obtained by PCR, in addition to the 485 bp band a ∼890 bp band was found. The 890 bp fragment was reamplified, cloned and sequenced.

### - Cloning and sequencing of the 890 bp LAG-3 fragment:

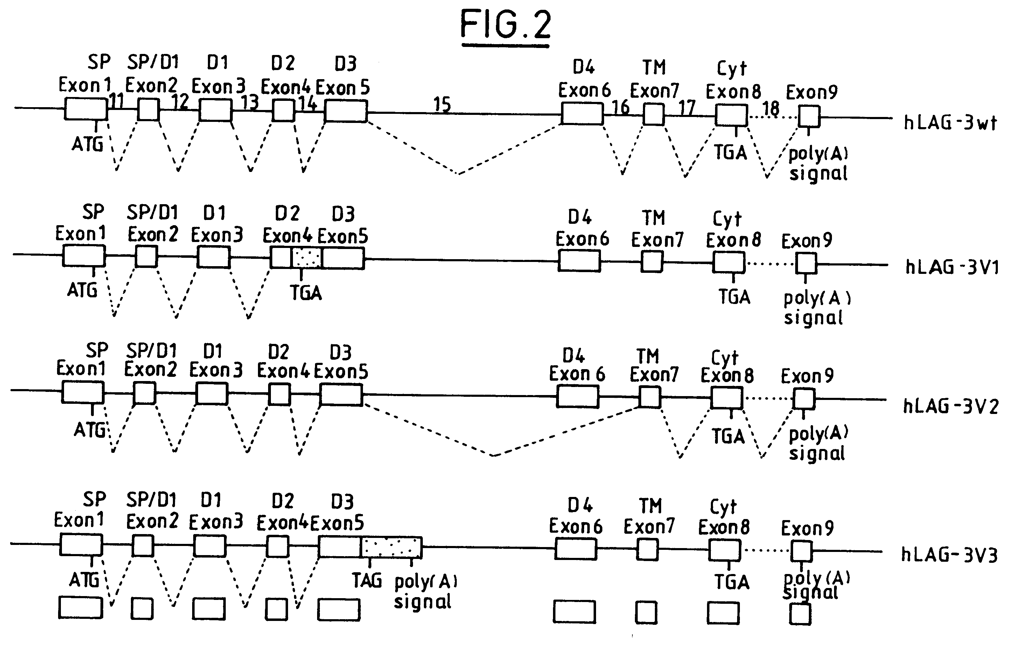

The 890 bp fragment was cloned into the vector pCR™II (Promega) and the insert was sequenced using the ABI Prism Dye Terminator Cycle Sequencing Ready Reaction Kit (Perkin-Elmer) and the automated DNA sequencer mod. 373A (Perkin-Elmer). The results indicate that this fragment is 892 bp in length and derives from a LAG-3 variant retaining intron 4 (Fig. 2). Due to the presence of an in-frame stop codon in intron 4, this variant, named LAG-3V1, is predicted to encode a soluble 36 kD protein containing domains D1, D2 and 8 new C-terminal amino acid residues (Fig. 1).

### - Confirmation of LAG-3V1 expression :

To confirm that the LAG-3V1 fragment derives from mRNA and not from genomic DNA, a second RT-PCR experiment was performed using the forward primer F176 (5' CCTGGGCCAGGCCTCGA TGAC 3') (SEQ ID N° 9) which anneals to nucleotides 725-745 of LAG-3 sequence, and the reverse primer R460 (Fig. 3). Primer F176 which spans the D1/D2 splice junction site, should not allow the amplification of LAG-3 genomic DNA.

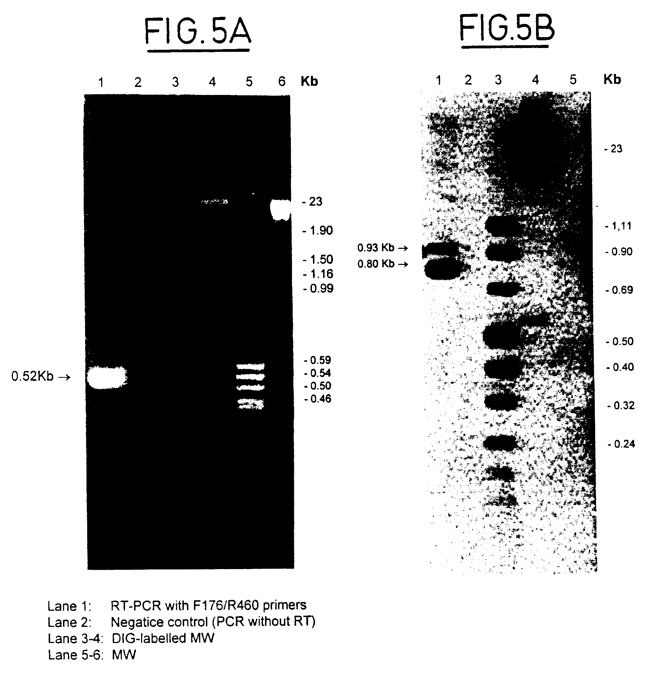

Upon agarose gel electrophoresis of the PCR products, the expected ∼520 bp wtLAG-3 band was observed (Fig. 5a). Southern blotting with the LAG-3 intron 4 specific oligoprobe I4 (5' CCCCACTCTGCTTCACATTT 3') (SEQ ID N° 10) showed the expected 930 bp LAG-3V1 band and an additional ∼800 bp band (Fig. 5b). The two fragments were reamplified and sequenced. The upper band was confirmed to correspond to LAG-3V1 while the lower one was a wtLAG-3/LAG-3V1 heteroduplex.

### Example II:

### Cloning of human LAG-3 variant 2 (LAG-3V2) and LAG-3 variant 3 (LAG-3V3) by RT-PCR on activated PBMC

### - RNA extraction and RT-PCR :

PBMC were isolated by Ficoll-Hypaque density gradient centrifugation and activated with 1 µg/ml PHA and 100 U/ml IL-2 for 48 hr at 37°C, 5% CO₂.

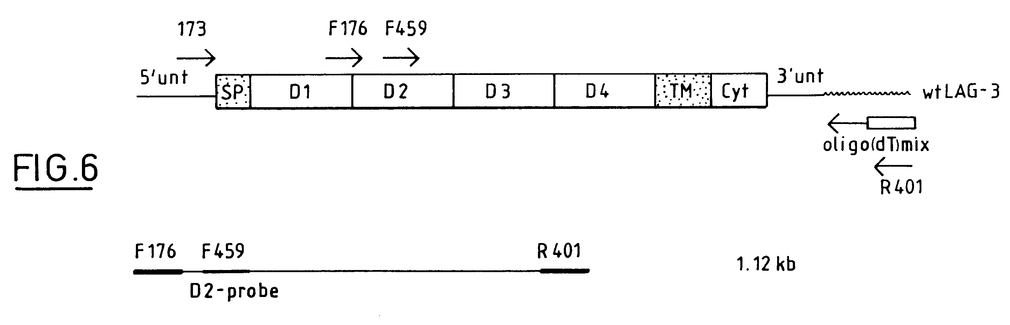

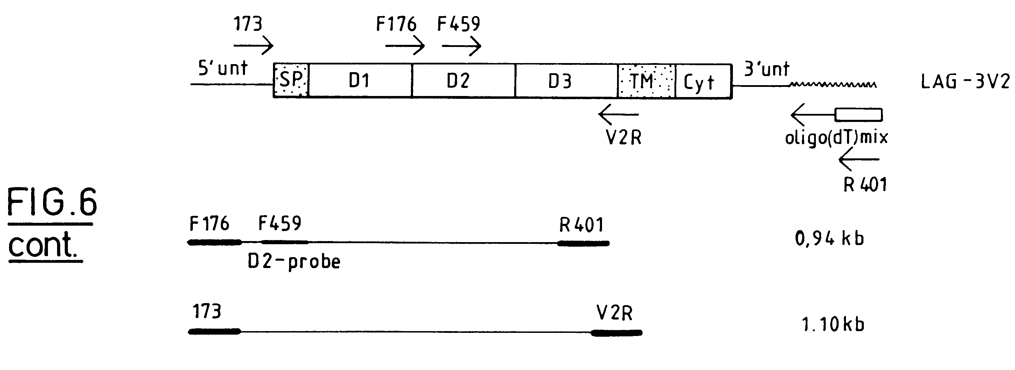

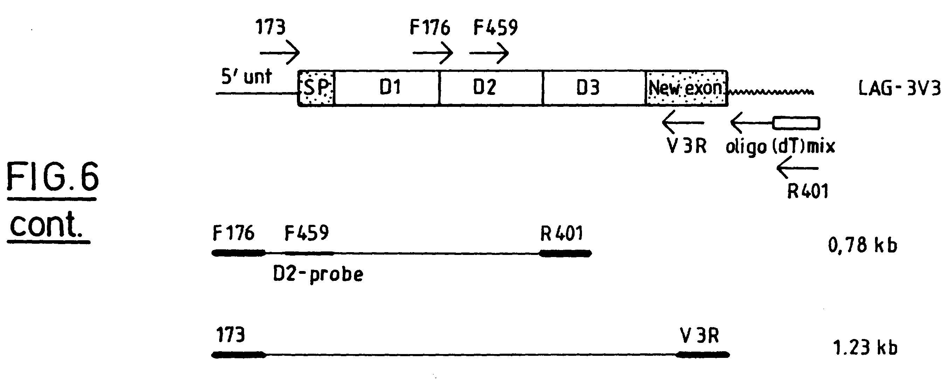

Total RNA was extracted by TriZol reagent (GIBCO-BRL). Total RNA (5µg) was reverse-transcribed using the anchor oligo(dT) primer mixture AT17A, AT17C and AT17G (5' GGCCCTGGATCCGGACCTAA(T)₁₇ (SEQ ID N° 1) followed by A, C or G) (Fig. 6) which allows to obtain cDNA molecules with homogeneous 3' ends and an anchor for subsequent PCR.

Reverse transriptase (RT) reaction was performed in 50 µl final volume of 1x RT buffer, 10 mM DTT, 1 mM dNTP, 40 U RNase inhibitor (Boehringer) and 400 U Superscript II RT (GIBCO-BRL) at 37°C for 90 min. RT reaction was stopped at 90°C for 5 min. RNA was digested with 1 U RNase H (Boehringer) at 37°C for 30 min.

PCR was performed with the forward primer F176 and the reverse primer R401 (5' GGCCCTGGATCCGGACCTAA 3') (SEQ ID N° 12) which anneals to the 3' anchor of cDNA (Fig. 6). Primers F176 and R401 should allow to amplify all LAG-3 splice variants containing the D1D2 junction up to poly(A) tail.

PCR was performed on 5 µl cDNA equivalent to 0.5 µg total RNA in 100 µl final volume containing 1x Taq buffer, 2.5 U Taq DNA polymerase (Advanced Biotechnologies), 1.5 mM MgCl₂, 200 µM dNTP, 10% DMSO and 50 pmoles each of F176 and R401 primers. A hot start technique and 30 PCR cycles (96°C for 30 sec, 65°C for 30 sec, 72°C for 4 min) were used. RNA without RT was used as negative control in PCR experiment.

### - Southern blot analysis of RT-PCR products :

RT-PCR products (10 µl) were fractionated by agarose gel electrophoresis and blotted onto Hybond N+ nylon membrane (Amersham). The blot was hybridized with the DIG-labeled LAG-3D2 specific oligoprobe F459 (Fig. 6) in 5X SSC, 0.02% SDS, 0.5% blocking reagent (Boehringer), 0.1% N-laurylsarcosine at 55 °C for 30 min.

The blot was washed twice with 2x SSC, 0.1% SDS at 55°C. Hybrid detection was performed with HRP-conjugated anti-DIG antibodies (Boehringer) and ECL reagents (Amersham).

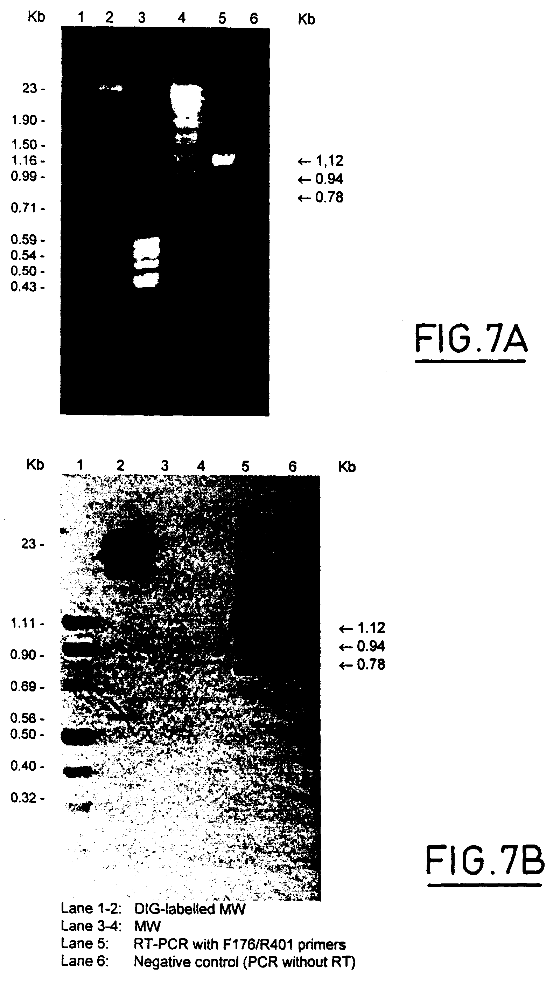

The results showed that in addition to the expected 1.12 kb wtLAG-3 fragment two minor bands of approximately 940 and 780 bp were present both in the ethidium bromide stained gel (Fig. 7a) and in the Southern blot (Fig. 7b).

### - Sequencing of LAG-3 fragments :

The 940 and 780 bp PCR fragments were isolated from agarose gel, reamplified with F176/R401 primers and directly sequenced. The results showed that the 780 bp fragment derives from an in-frame skipping of LAG-3 exon 6 (Fig. 2). This variant, named LAG-3V2, is predicted to encode a 61 kD transmembrane protein which does not contain the domain D4 (Fig. 1).

Sequencing of the 940 bp fragment showed that it derives from the cleavage of the nuclear transcript at a different polyadenylation site located ∼170 bp downstream to the 5' end of intron 5 (Fig. 2). The retained intron 5 sequence contains an in-frame stop codon. The resulting mRNA encodes a 52 kD soluble LAG-3 variant, named LAG-3V3, which contains domain D1, D2 and D3 followed by 8 new amino acid residues (Fig. 1). Both LAG-3V2 and LAG-3V3 maintain the 4 glycosylation sites of wtLAG-3.

### - Confirmation of LAG-3V2 and LAG-3V3 expression :

To confirm the expression of LAG-3V2 and LAG-3V3 and to assess the presence of the entire 5' sequence encoding the LAG-3 N-terminus, RT-PCR was performed on total RNA from activated PBMC with the forward primer 173 (5' TATAGGATCCGGTGCCCAG ACCATAGGAGAGATG 3') (SEQ ID N° 13) which anneals to nucleotides 212-233 of LAG-3 sequence spanning the ATG start codon, and the reverse primers V2R (5'GGCGTTCACGTGGTTGGGCACCTGTGATGATT 3') (SEQ ID N° 14) or V3R (5'TCACCTACTCGAGAAAAGTGGGGGCCGAGAT 3') (SEQ ID N° 15) (Fig. 6). As the primer V2R anneals to the splice junction site D3/TM of LAG-3V2 and the primer V3R anneals to the 5' sequence of intron 5 retained in LAG-3V3, only amplification of these two variants should occur.

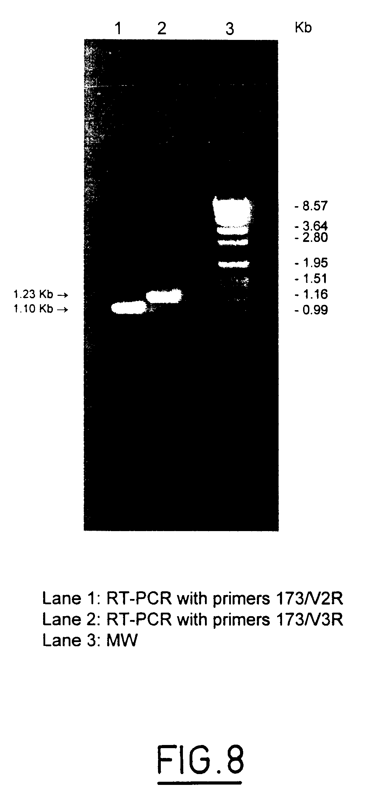

PCR was performed with annealing temperature at 68°C for the first two cycles and 72°C for the remaining 28 cycles. Upon agarose gel electrophoresis, the expected 1.10 Kb LAG-3V2 and 1.23 Kb LAG-3V3 fragments were found (Fig. 8). DNA sequencing of the amplified fragments confirmed that LAG-3V2 encodes a 61 kD transmembrane protein containing domains D1, D2, D3, the transmembrane domain (TM) and the cytoplasmic domain (CYT), while LAG-3V3 encodes a 52 kD soluble protein containing D1, D2 and a new 8 aa C-tail (Fig. 1).

In conclusion, at present four LAG-3 molecules have been discovered, two membrane-bound (wtLAG-3 and LAG-3V2) and two soluble forms which lack the transmembrane sequence (LAG-3V1 and LAG-3V3). The analysis of LAG-3 variant expression in specific T cell subsets and in different activation states can provide useful information for a better understanding of the LAG-3 function

### Example III:

### Characterization of LAG-3V3 protein

Human peripheral blood mononuclear cells (PBMC) were obtained by centrifugation on ficoll-diatrizoate density gradient from buffy coats of blood donation units. PBMC were obtained from two healthy donors. PBMC collected from the interface between the supernatant and the gradient were washed three times in PBS and finally resuspended at the concentration of 2.10⁶ cells/ml in RPMI 1640 culture medium enriched with 10 % fetal bovine serum (FBS), 2 mM L-glutamine, penicillin 100 IU/ml and streptomycin 100 µg/ml. Phytohemagglutinin (PHA) and recombinant human interleukin (IL-2) were then added at a final concentration of 1 µg/ml and 100 U/ml respectively.

The cells were incubated at 37°C in 5 % CO₂ atmosphere for 72 and 120 hours. At the end of the incubation times, cell suspensions were transferred to 50 ml polypropylene tubes and centrifuged at 400 g for 10 minutes. The supernatants were collected and immediately frozen at -20°C. The total protein determination was carried out by the Bradford method, using the Coomassie Plus Protein Assay (Pierce, Rockford, IL, USA). For analysis, 100 µg of samples were completely dried under vacuum centrifugation, resuspended in 10 µl bi-distilled water (ddH₂O), 10 µl sample buffer 2x were added and the samples were incubated 5 minutes at 100°C.

The samples were then loaded on 8 % polyacrylamide Tris glycine gels for SDS-PAGE analysis and run under denaturating and reducing conditions at a constant 125 volts. The run was stopped when the dye front reached the bottom of the gel.

The gel was removed and incubated for 20 minutes in transfer buffer. A nitrocellulose membrane 0.45 µm was incubated for 10 minutes in transfer buffer in a separate container with gentle agitation. The proteins in the gel were then transferred electrophoretically to the membrane for 90 minutes at a constant 75 volts. When the transfer was completed, the membrane was dried at room temperature and then incubated for 20 minutes in 1 % (w/v)KOH with gentle agitation and washed 4 times with PBS for 5 minutes. The membrane was stained 5 minutes in Ponceau S solution to show proteins and molecular weight markers and destained 4 times with PBS. Non-specific binding sites were blocked by incubating the membrane overnight at 4°C with 1 % Mile in PBS/0.5 % Tween. After elimination of blocking solution, the membrane was blotted with anti-LAG-3 monoclonal antibody (mAb) 2H6 diluted at 5 µg/ml blocking solution. After one hour of incubation at room temperature with agitation, the membrane was washed 5 times with PBS/0.5 % Tween for 10 minutes each and than incubated for one hour at room temperature in agitation with goat anti-mouse polyclonal antibodies conjugated with horseradish peroxidase (HRP)diluted 1:1000 in blocking solution. At the end of the incubation, the membrane was washed as described above.

### RESULTS

The immunoblotted proteins were then revealed using the enhanced-chemiluminescent (ECL) method (Amersham Italia, Milan Italy).

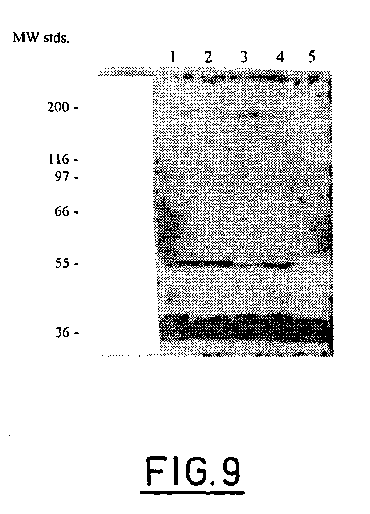

Lanes 1 and 3 of figure 9 represent the results obtained from donor 1, respectively after 72 and 120 hours incubation ; whereas lanes 2 and 4 correspond to donor 2 and lane 5 to the lymphoblastoid cell line RAJI as negative control. The starting protein concentrations were respectively 3.1 mg/ml; 1.9 mg/ml; 2.8 mg/ml; 3.4 mg/ml; 3.4 mg/ml from lane 1 to lane 5.

A band with an apparent molecular weight of about 5.5 kDa reacted with anti-LAG-3 mAb. This apparent molecular weight is in agreement with that expected for the putative LAG-3 variant 3 based on the mRNA sequence. This band specifically appears in PHA-blasts and is not present in the supernatant of the B-Iymphoblastic cell line RAJI, processed as the supernatants from PHA-blasts.

## Claims

1. An isolated nucleotidic sequence selected from the group consisting of :
a) the nucleotidic sequences SEQ ID N° 1, SEQ ID N°3 or SEQ ID N°5 ;
b) the nucleotidic sequences which hybridize under stringent conditions to any of the sequences defined in a) and which code for a polypeptide which is a variant of the LAG-3 molecule ;
c) the nucleotidic sequences which are degenerated as a result of the genetic code to the nucleotidic sequences defined in a) and b), and which code for a polypeptide which is a genetic variant of the LAG-3 molecule.

2. A nucleotidic sequence according to claim 1 selected from the group consisting of the nucleotidic sequences SEQ ID N° 1, SEQ ID N° 3, SEQ ID N° 5 or the fully complementary sequences thereof.

3. A purified polypeptide encoded by a nucleotidic sequence according to claim 1 or 2.

4. A polypeptide according to claim 3, selected from the group consisting of LAG-3V1, LAG-3V2 and LAG-3V3, having a sequence selected from the group consisting of SEQ ID N° 2, SEQ ID N° 4 and SEQ ID N°6 respectively encoded by SEQ ID N° 1, SEQ ID N° 3 or SEQ ID N° 5.

5. An expression vector comprising a nucleotidic sequence according to claim 1 or 2.

6. An host cell transformed with an expression vector according to claim 5.

7. A pharmaceutical composition containing as active ingredient a polypeptide according to claim 3 or 4.

8. Antibodies directed to a specific epitope of one of the polypeptides according to claim 3 or 4.

9. Antibodies according to claim 8, wherein said antibodies are monoclonal antibodies or Fab, Fab', F(ab') or Fv fragments thereof.

10. Use of antibodies according to claim 8 or 9 in a method for purifying, dosing or identifying a polypeptide according to claim 3 or 4.

11. Use of antibodies according to claim 8 or 9 for the manufacture of a therapeutic composition for treating immune-related pathologies.

12. A therapeutic composition comprising as active ingredient an antibody according to claim 8 or 9.

13. Use of a polypeptide according to claim 3 or 4 for the manufacture of immunomodulators compounds.

14. Use of a polypeptide according to claim 3 or 4 for the manufacture of a therapeutic composition for treating treating immunerelated pathologies.
